# EUROPEAN PATENT APPLICATION

(11) **EP 0 811 693 A2**
(43) Date of publication of application: **10.12.1997**
(21) Application number: 97108778.8
(22) Date of filing: 02.06.1997
(51) Int. Cl.: C12Q 1/68

(54) **A method of determining intracellular telomerase activity**

(30) Priority: 03.06.1996 JP 140534/96; 27.05.1997 JP 136959/97
(71) Applicant: KYOWA HAKKO KOGYO CO., LTD., Chiyoda-ku, Tokyo-to (JP)
(72) Inventor: Ohyashiki, Kazuma, Tokyo-to (JP); Ohyashiki, Junko, Tokyo-to (JP)
(74) Representative: VOSSIUS & PARTNER

(57) **Abstract**

There is disclosed a method of determining telomerase activity comprising:
(i) a step of immobilizing a sample on a slide glass;
(ii) a step of conducting elongation reaction of telomerase substrate with telomerase contained in said sample while said sample being immobilized on said slide glass; and
(iii) a step of subjecting the resulting elongation reaction product to base binding chain reaction while said sample being immobilized on said slide glass.

## Description

The present invention relates to a method of determining the activity of intracellular telomerase which plays an important role in division and proliferation of cancer cells and which is an important approach to be determined in a clinical laboratory test, to a method of pathological examination and to a reagent for determining such enzyme activity. The present invention is significant in clinical laboratory testing technology as well as pathological diagnosing technology, for example, for diagnosing cancer.

The method of determining telomerase activity is proceeded by the following steps. A radioisotope labeled primer is subjected to telomerase reaction, and, after amplifying the telomerase reaction product by polymerase chain reaction (PCR), the PCR reaction product is subjected to electrophoresis followed by autoradiography, thereby determining the activity (Kim, et al., Science, 266, 2011 (1994); WO 95/13381).

Since the sample for the methods of the prior art is restricted to the cell extract etc., the methods of the prior art can not detect individual cellular telomerase activity. Therefore, the methods of the prior art can not be applied to a diagnosis at a cellular level, employed frequently in diagnosing cancers.

Accordingly, a main object of the present invention is to provide a simple method in determining telomerase activity which is applicable to the diagnosis at the cellular level for the purpose of early detection of cancers.

This object as well as other objects and advantages of the present invention will become apparent to those skilled in the art from the following description.

In order to solve the problems mentioned above, a unique method is provided wherein the telomerase reaction is conducted using a fluorescent-labeled substrate on a slide glass employed in the diagnosis at the cellular level and then a fluorescent-labeled primer is subjected to base binding chain reaction to generate an intracellular telomerase reaction-induced fluorescent light, which is identified by gross observation by a fluorescent microscope.

Accordingly, the present invention relates to a method of determining telomerase activity comprising:
(i) a step of immobilizing a sample on a slide glass;
(ii) a step of conducting elongation reaction of telomerase substrate with telomerase contained in said sample while said sample being immobilized on said slide glass; and
(iii) a step of subjecting the resulting elongation reaction product to base binding chain reaction while said sample being immobilized on said slide glass.

The step of immobilizing a sample on a slide glass may be a step of adding the sample on the slide glass followed by drying followed by immobilization or a step of placing a freeze-dried section preparation on the slide glass which is then immobilized.

The slide glass may be a slide glass employed generally in microscope observation. Although a non-fluorescent slide glass is preferable, a silane-coated slide glass is more preferable and a non-fluorescent silane-coated glass is most preferable.

The sample may be any material which is derived from viable organisms and which may be subjected to clinical tests, such as blood, lymph fluid, urine, secretions, tissue section and the like, while the cells isolated from blood or fluid obtained by puncture are preferable.

The sample may be added to the slide glass by dispensing with pipettes or by using automatic cell collectors such as CYTOSPIN (K. Inoue, et al., Guidelines for diagnosis at cellular level (Ed. by S. Inoue, N. Nakamura), page 56, Published by TOSSHOKANKOKAI of Hokkaido Univ., (1995)). When a tissue section is employed as the sample, the freeze-dried piece is prepared in a usual manner and then placed on a slide glass (K. Katsuyama, et al., Outlines of clinical laboratory testings (Ed. by M. Kanai), page 1213, Published by KINBARASHUPPAN, (1995)).

When the sample is immobilized on the slide glass, it is preferable to dry with cool air to solidify the sample immediately after placing the sample, but it may be possible to immobilize the sample using a cell immobilizing agent containing, for example, polyethylene glycol. One of commercial cell immobilizing agents is CYTOKEEP (Tradename: Manufactured by NIPPON SHOJI).

To conduct the elongation reaction of the telomerase substrate by the telomerase contained in the sample being immobilized on the slide glass means that the telomerase substrate elongation reaction is effected by adding a fluorescent-labeled form of the telomerase substrate and deoxynucleoside triphosphates to the sample on the slide glass obtained in step (i).

The telomerase according to the present invention (EC 2.7.7.31) means an enzyme involved in synthesis of repeating sequence of telomere (telomere repeating sequence) which is the terminal structure essential for a linear chromosome having self-repair functions. The telomere means a terminal moiety of a straight chain chromosome and the telomere repeating sequence means a repeating sequence of a certain base sequence localized in the telomere.

The telomerase substrate is preferably an oligonucleotide containing no telomeric sequence and may be 5'-GTTAGGGTTAGGGTTAGG-3', 5'-TTAGGGTTAGGGTTAGGG-3' and the like, although the most preferable substrate is TS primer (5'-AATCCGTCGAGCAGAGTT-3').

The fluorescent-labeled form of the telomerase substrate means a telomerase substrate containing a telomerase substrate bound to a fluorescent label. The telomerase substrate bound to the fluorescent label can be obtained by binding a fluorescent reagent such as fluorescent phosphoramidite (Manufactured by CLONETECH) to the telomerase substrate (Makino, et al., PCR methods and applications (1992), Published by COLD SPRING HARBOR LABORATORIES).

The deoxynucleoside triphosphates mean the mixtures of deoxynucleoside triphosphates and consist of deoxyadenosine triphosphate, deoxyguanosine triphosphate, deoxycytidine triphosphate and deoxythymidine triphosphate.

The telomerase substrate elongation reaction means a reaction in which a telomere repeating sequence originating from the fluorescent substance of the telomerase substrate is synthesized by the telomerase contained in the sample. More particularly, according to the telomerase repeating sequence, various deoxynucleoside triphosphates such as deoxyadenosine triphosphate are bound to the fluorescent-labeled form of the telomerase substrate by the catalysis of the telomerase contained in the sample. As the result of the enzyme reaction, the elongated form of the telomerase substrate is synthesized.

The telomerase substrate elongation reaction is found in the literature by LITE (LITE, et al., NUCLEOSIDES AND RESEARCH 23, 3794 (1955)). The present invention is carried out by the modification method of Lite. The modification method is considered that the reaction must be performed on the slide glass. Thus, after adding a fluorescent-labeled form of the telomerase substrate and a buffer solution containing deoxynucleoside triphosphates on the slide glass, the sample is covered and incubated. In this step, it is preferable to supplement the buffer solution with either a heat-resistant (Taq) base binding enzyme for the base binding enzymatic chain reaction or a primer. The incubation is conducted preferably in a chamber protected from light.

The buffer solution may be any of those at a concentration of 1 to 100 mM and at a pH of 6 to 9, preferably 7.5 to 8.5, although phosphate buffers and Tris-HCl buffers are preferred, such buffer solutions may further contain inorganic salts such as potassium chloride, calcium chloride, sodium chloride and magnesium chloride, surfactants such as polyoxyethylene sorbitan monolaurate (hereinafter referred to as Tween 20), chelating agents such as ethylene glycol bis(2-aminoethylethyl)-N,N,N',N'-tetraacetic acid (hereinafter referred to as EGTA) as well as proteins such as T4 gene 32 protein and bovine serum albumin.

To cover the sample means to cover the sample on the slide glass with a cover glass or paraffin films and the like.

In cases where PCR is employed for the base binding enzymatic chain reaction, the primer may be any of those constructed from incomplete telomeric repeating sequences (5'-CCCTTA-3') and complete telomeric repeating sequences (5'-CCCTAA-3'), and it is preferable to employ a fluorescent-labeled CX reverse primer constructed from three incomplete telomeric repeating sequences and one complete telomeric repeating sequence (5'-CCCTTACCCTTACCCTTACCCTAA-3'). In cases where ligase chain reaction (LCR) is employed for the base binding enzymatic chain reaction, any of those constructed from C-rich telomeric sequences (5'-CTAACC-3') may be employed, and it is preferable to employ a fluorescent-labeled ACT primer (5'-GCGCGGCTAACCCTAACCCTAACC-3').

Such primers as well as the following oligonucleotide primers described in the present invention can be obtained by a standard manner using an automatic DNA synthesizer.

The fluorescent-labeled CX reverse primer or fluorescent-labeled ACT primer means a CX reverse primer or ACT primer bound to a fluorescent label, respectively. The CX reverse primer or ACT primer bound to a fluorescent label can be obtained by binding a fluorescent reagent such as fluorescent phosphoramidite (Manufactured by CLONETECH) to the CX reverse primer or ACT primer (Makino, et al., PCR method and applications (1992), Published by COLD SPRING HARBOR LABORATORIES).

The heat-resistant (Taq) base binding enzyme represents a Taq DNA polymerase (E.C.2.7.7.7) when employing PCR in the base binding enzymatic chain reaction and a Taq DNA ligase (E.C.6.5.1.1) when employing LCR.

Subjecting the resulting elongation reaction product to base binding chain reaction while said sample being immobilized on said slide glass means that a primer when a heat resistant base enzyme has already been added in step (ii) above or a heat resistant base enzyme when a primer has already been added in the same step is added to the sample on the slide glass which has already been
subjected to step (ii), thereby mixing the base binding enzyme, the telomerase substrate elongation reaction product obtained in step (ii) and the primer to achieve the base binding enzymatic chain reaction.

The base binding enzymatic chain reaction may be PCR and LCR, based on the selection from which the heat resistant base binding enzyme and the primer are determined as mentioned above.

More particularly, the telomerase substrate elongation reaction product and the primer may be subjected to the PCR reaction to amplify using the Taq DNA polymerase (SAIKI, et al., Science 239, 487 (1988); Kim, et al., Science 266, 2011 (1994)), or , alternatively, the telomerase substrate elongation reaction product and the primer may be subjected to the LCR reaction to amplify using the Taq DNA ligase (BALANEY, et al., Proc. Natl. Acad. Sci., 87 1874 (1987)).

By combining the heat resistant base binding enzyme and the primer appropriately as described above, the base binding enzymatic chain reaction is initiated, and, in order to conduct this reaction on a slide glass, it is preferable to employ, for example, a commercially available automatic PCR system (Tradename: OMNISLIDE, manufactured by HYBAID LTD).

After completion of the base binding enzymatic chain reaction followed by washing the slide glass with a washing fluid, the telomerase activity in the sample can be detected using a fluorescent microscope. As the washing fluid, any fluids such as physiological saline and purified water may be employed. It is preferable to enclose the sample with the mixture of MACLVAINE buffer and glycerin. Any commercial fluorescent microscopes may be employed.

The present invention is further described in detail below.

From a sample obtained from viable organisms such as blood, cells are harvested in a standard manner using, for example, centrifugation. After washing the cells harvested with a cell culture medium or equivalent, the cells were allowed to stand on a non-fluorescent slide glass using a cell dispenser such as CYTOSPIN (K. Inoue, et al., Guidelines for diagnosis at cellular level (Ed. by S. Inoue, N. Nakamura), page 56, Published by TOSSHOKANKOKAI of Hokkaido Univ., (1995)). The sample is air-dried and immobilized.

1 to 100 mM Tris-HCl buffer (pH 6 to 9) is supplemented with 1 to 1000 pmol fluorescent-labeled TS primer (5'-AATCCGTCGAGCAGAGTT-3'), 1 to 300 mM deoxynucleoside triphosphates and 0.1 to 20 units of Taq DNA polymerase are added to the sample on the slide glass. The slide glass is covered with a paraffin film, and incubated at a temperature of 10 to 50 °C, preferably 20 to 40 °C, more preferably at 25 °C, for a period of 7 minutes to 1 hour 30 minutes, preferably 10 minutes to 1 hour, more preferably 30 minutes to 1 hour, the most preferably for 1 hour in a chamber protected from light.

After completion of the TS primer elongation reaction, 1 to 300 pmol, preferably 50 to 200 pmol, more preferably 100 pmol fluorescent-labeled CX reverse primer (5'-CCCTTACCCTTACCCTTACCCTAA-3') is added, thereby initiating PCR, in which a thermal reaction cycle of PCR may be, for example, 1 to 120 seconds at 90 to 100 °C, 1 to 120 seconds at 40 to 60 °C, and 1 to 240 seconds at 60 to 80 °C, which are repeated 1 to 50 times. Since PCR is conducted on the slide glass, the OMNISLIDE mentioned above or equivalent is preferably employed.

After completion of PCR and washing the slide glass with the washing fluid, the telomerase activity in the sample can be identified using a fluorescent microscope.

The telomerase activity assay reagent according to the present invention may be combined with fluorescent-labeled CX reverse primers, deoxynucleoside triphosphates, fluorescent-labeled TS primers and Taq DNA polymerases, as well as slide glasses, sample immobilizing devices and T4 gene 32 proteins. In addition, the buffer solutions mentioned above, washing fluids and various additives thereto as listed above may also be combined.

The following Examples further illustrate the present invention in detail but are not to be construed to limit the scope thereof.

### Example 1

HEL cells were harvested. The cells harvested were washed with phosphate buffer (pH 6.4) and then attached to a non-fluorescent slide glass using CYTOSPIN (500 rpm, 3 minutes). The sample was air-dried immediately, and immobilized.

25 µl of 20 mM Tris-HCl buffer (pH 6 to 9) [containing 1.5 mM magnesium chloride, 63 mM potassium chloride, 0.05% Tween 20 (Tradename), 1 µM EGTA, 1 µg/ml T4 gene 32 protein (Manufactured by Boehringer Mannheim), bovine serum albumin (0.1 mg/ml)] was supplemented with 100 pmol fluorescent-labeled TS primer (5'-AATCCGTCGAGCAGAGTT-3') [Manufactured by PHARMABIOTECH], 50 mM deoxynucleoside triphosphates and 2 units of a Taq DNA polymerase, and added to the sample on the slide glass. The slide glass was covered with a paraffin film, and incubated at 25 °C for 30 minutes in a chamber protected from light.

After completion of the TS primer elongation reaction, 25 µl of 100 pmol fluorescent-labeled CX reverse primer (5'-CCCTTACCCTTACCCTTACCCTAA-3') was added to the sample on the slide glass. Upon this addition, PCR was initiated and PCR was conducted in OMNISLIDE mentioned above. The thermal cycles were 30 seconds at 94 °C, 30 seconds at 50 °C and 90 seconds at 72 °C, which were repeated 30 times.

After completion of PCR and washing the slide glass with spouting water, the sample was embedded in MACLVAINE buffer/glycerin solution (1:1=v:v). The labeled cells were observed by a fluorescent microscope using a G filter and the telomerase activity in the sample was identified.

To examine whether the fluorescent signals derived from HEL cells which were identified microscopically were dependent on the telomerase activity or not, cell membranes were broken after microscopic observation, and the effluent was subjected to gel electrophoresis and then injected to a 6% acrylamide gel containing 6 M urea (Tradename: LONGRANGER: Manufactured by AT BIOCHEM), which was subjected to electrophoresis. The fluorescent bands in the gel were analyzed automatically using a fragment manager program (Produced by Pharmacia BIOTECH) (WO 95/13381).

Intermittent peaks of 6 base pairs attributable to the telomerase activity were observed. Thus, the cellular fluorescent peaks observed in the assay of the intracellular telomerase activity described above were confirmed to be attributable to the PCR products synthesized by the intracellular telomerase. Further, the samples treated similarly except for omitting only PCR treatment or except for omitting only the TS primer elongation reaction exhibited attenuated fluorescence, supporting that the fluorescent peaks were attributable to the PCR products synthesized by the intracellular telomerase.

Based on the results described above, it was proven that the telomerase activity in cells and tissues can be determined conveniently by the intracellular telomerase assay method according to the present invention.

### Example 2

In order to determine telomerase activities in various cancer cells shown in Table 1, a population of 500 cells of each cancer was employed as a sample, which was subjected to the intracellular telomerase activity assay according to the method in Example 1. In microscopic observation, the cellular fluorescent intensity was evaluated as one of the 3 degrees, namely, markedly positive, positive and negative, and the cells exhibiting such degrees within the vision were counted to obtain % positive as the ratio based on the total cell counts within the vision.

The results are shown in Table 1.

**Table 1**

| | Percent positive (%) | | |
|---|---|---|---|
| | Negative | Positive | Markedly positive |
| Leukemia/lymphoma cell lines | | | |
| Erythroblastic HEL | 0.2 | 0.4 | 99.4 |

| Megakaryoblastic | | | |
|---|---|---|---|
| YS9;22 | 0.6 | 26.4 | 72.6 |
| TS9;22 | 0.4 | 4.6 | 95.0 |
| SS9;22 | 0.0 | 0.2 | 99.8 |
| MOLM-1 | 0.6 | 9.6 | 89.8 |
| Myeloblastic HL60* | 5.6 | 18.6 | 75.8 |
| Histiocytic U937 | 0.0 | 0.4 | 99.6 |

| Lymphoblastic | | | |
|---|---|---|---|
| OM9;22 | 3.8 | 0.0 | 96.2 |
| HAL-01 | 0.0 | 2.6 | 97.4 |
| TALL-2 | 9.0 | 0.2 | 90.8 |

| Solid-tumor cell lines | | | |
|---|---|---|---|
| KLE | 0.0 | 0.4 | 99.6 |
| Naucc-1 | 0.0 | 0.6 | 99.4 |
| Naucc-3 | 0.0 | 96.6 | 3.4 |
| Caski | 0.0 | 8.4 | 91.6 |
| SCH | 0.0 | 2.2 | 97.8 |
| JEG-3 | 0.0 | 99.6 | 0.4 |
| CA-99 | 0.0 | 0.0 | 100.0 |
| Kawauchi | 0.0 | 4.2 | 95.8 |
| JAR | 0.0 | 0.0 | 100.0 |
| SKG-I | 0.0 | 17.2 | 82.8 |
| SKG-II | 0.0 | 0.0 | 100.0 |
| SKG-IIa | 0.0 | 0.0 | 100.0(50.6) |
| SKG-IIIb | 0.0 | 0.0 | 100.0(3.8) |
| MEC-I | 0.0 | 0.0 | 100.0(26.6) |
| Hela | 0.0 | 0.2 | 98.8 |

### Example 3

Telomerase activity percent positives and fluorescent intensities of the cells in the vision were determined according to the same manners as those in Examples 1 and 2 except that K562 cells were used instead of HEL cells and incubation was conducted for 15 minutes, 30 minutes and 1 hour in a chamber protected from light.

The results are shown in Table 2.

**Table 2**

| Incubation time | Percent positive (%) | Fluorescent intensity |
|---|---|---|
| 15 min. | 26 | Weak |
| 30 min. | 42 | Strong |
| 1 hour | 44 | Strong |

As described above, according to the present invention, a method and reagent useful in determining the telomerase activity which is essential for clinical diagnosis is provided. Since the method according to the invention is convenient and applicable to the diagnosis of cancer cells which is conducted frequently in the field of clinical medicine, it can be utilized in clinical laboratory tests, pathological tests and the like.

Sequencing listing

Sequence ID No.: 1

Length: 18

Type: Nucleic acid

Strandedness: Single-stranded

Topology: Linear

Molecular type: Other nucleic acid

Sequence description: GTTAGGGTTA GGGTTAGG

Sequence ID No.: 2

Length: 18

Type: Nucleic acid

Strandedness: Single-stranded

Topology: Linear

Molecular type: Other nucleic acid

Sequence description: TTAGGGTTAG GGTTAGGG

Sequence ID No.: 3

Length: 18

Type: Nucleic acid

Strandedness: Single-stranded

Topology: Linear

Molecular type: Other nucleic acid

Sequence description: AATCCGTCGA GCAGAGTT

Sequence ID No.: 4

Length: 24

Type: Nucleic acid

Strandedness: Single-stranded

Topology: Linear

Molecular type: Other nucleic acid
Sequence description: CCCTTACCCT TACCCTTACC CTAA
Sequence ID No.: 5
Length: 24
Type: Nucleic acid
Strandedness: Single-stranded
Topology: Linear
Molecular type: Other nucleic acid
Sequence description: GCGCGGCTAA CCCTAACCCT AACC

## Claims

1. A method of determining telomerase activity comprising:
(i) a step of immobilizing a sample on a slide glass;
(ii) a step of conducting elongation reaction of telomerase substrate with telomerase contained in said sample while said sample being immobilized on said slide glass; and
(iii) a step of subjecting the resulting elongation reaction product to base binding chain reaction while said sample being immobilized on said slide glass.

2. The method according to claim 1, wherein the step of immobilizing a sample on a slide glass comprises a step of drying with a cool air to solidify the sample immediately after placing the sample on the slide glass.

3. The method according to claim 1 or 2, wherein the step of conducting elongation reaction of telomerase substrate with telomerase contained in said sample while said sample being immobilized on said slide glass comprises a step of adding a fluorescent-labeled form of the telomerase substrate and deoxynucleoside triphosphates to the sample on the slide glass and conducting incubation in a chamber protected from light.

4. The method according to any one of claims 1-3, wherein the step of subjecting the resulting elongation reaction product to base binding chain reaction while said sample being immobilized on said slide glass comprises a step of adding a heat resistant base enzyme and a primer to the sample on the slide glass to mix a base binding enzyme, the telomerase substrate elongation reaction product and the primer and, whereby, the base binding enzymatic chain reaction is achieved.

5. The method according to any one of claims 1-4, wherein the method of determining telomerase activity is by gross observation using a fluorescent microscope.

6. A telomerase activity assay kit which comprises a fluorescent-labeled CX reverse primer, a fluorescent-labeled TS primer, a Taq DNA polymerase, a slide glass and a sample immobilizing device.
